# EUROPEAN PATENT APPLICATION

(11) **EP 2 821 398 A1**
(43) Date of publication of application: **07.01.2015**
(21) Application number: 13174887.3
(22) Date of filing: 03.07.2013
(51) Int. Cl.: C07D 213/80, C07D 213/803, C07C 227/04, C07C 229/30, C07D 211/90

(54) **Method for preparation of 6-trifluoromethylpyridine-3-carboxylic acid derivatives from 4,4,4-trifluoro-3-aminobutanoates**

(71) Applicant: Lonza Ltd, 3930 Visp (CH)
(72) Inventor: Zaragoza Dörwald, Florencio, 3930 Visp (CH); Täschler, Christoph, 3930 Visp (CH); Bersier, Michael, 3938 Ausserberg (CH)

(57) **Abstract**

The invention discloses a method for the preparation of substituted 6-trifluoromethylpyridine-3-carboxylic acid derivatives starting from 4,4,4-trifluoro-3-aminobutanoates and proceeding via enamines and dihydropyridinones.

## Description

The invention discloses a method for the preparation of substituted 6-trifluoromethylpyridine-3-carboxylic acid derivatives starting from 4,4,4-trifluoro-3-aminobutanoates and proceeding via enamines and dihydropyridinones.

### BACKGROUND OF THE INVENTION

2-Trifluoromethylpyridines and 6-trifluoromethylpyridine-3-carboxylic acid derivatives are intermediates for the preparation of biologically active compounds. For instance, WO 00/39094 A1 discloses trifluoromethylpyridine as herbicides, WO 2006/059103 A2 discloses trifluoromethylpyridines as intermediates in the production of pharmaceutical, chemical and agro-chemical products, WO 2008/013414 A1 discloses trifluoromethylpyridines as vanilloid receptor antagonists and WO 2012/061926 A1 describes trifluoromethylpyridines as calcium channel blockers.

The common route for the preparation of 6-trifluoromethylpyridine-3-carboxylic acid derivatives was first reported by Okada et al., Heterocycles 1997, 46, 129-132, and has only been slightly modified by others. The common synthetic strategies are summarized in Scheme 1:

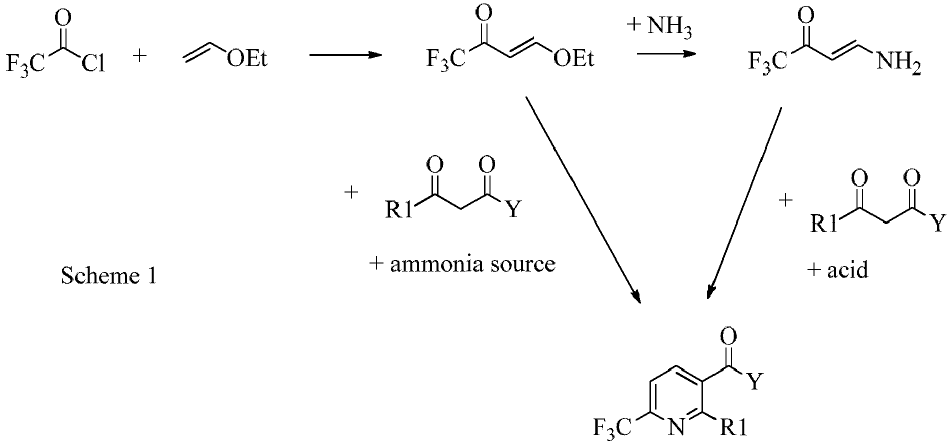

This route has disadvantages for the large scale production of 6-trifluoromethylpyridine-3-carboxylic acid derivatives, because ethylvinylether is highly flammable and therefore difficult to handle, and because the trifluoroacetylated enolether and the trifluoroacetylated enamine intermediates are unstable and cannot be stored for a longer time. Moreover, most vinyl ethers are mutagenic.

Cen et al. Journal of Fluorine Chemistry 1995, 73, 161-164 discloses certain precursors used in instant invention.

D. Ma et al. in Org. Lett. 2000, 2, 2503-2505 and N. Leflemme et al. in Synthesis 2002, 12, 1740-1746 disclose the preparation of dihydropyridones from enamines.

EP 448542 A2 discloses that trifluoromethyl residues can suffer elimination of HF, i.e. dehydrofluorination, upon treatment with bases as depicted in Scheme I and Scheme II of EP 448542 A2.

There was a need for an improved procedure for the preparation of 6-trifluoromethylpyridine-3-carboxylic acid derivatives.
This need was met by the method of instant invention as outlined below.
Compared to prior art, the method of the instant invention offers several advantages: Importantly, no vinyl ethers, trifluoroacetylated enolether intermediates or trifluoroacetylated enamine intermediates are required. Moreover, the method of the present invention only comprises few synthetic steps, what reduces the overall costs.
Unexpectedly and against the teaching of EP 448542 A2, enamines carrying trifluoromethyl residues could be cyclized to 2,3-dihydropyridin-4(1H)-ones without elimination of HF. Moreover, a method was found for the unprecedented dehydratization of 2,3-dihydropyridin-4(1H)-ones to pyridines.

In the following text, if not otherwise stated, the following meanings are used:
- ambient pressure: usually 1 bar, depending on the weather;
- alkyl: means a linear or branched alkyl, examples of alkyl include methyl, ethyl, n-propyl, isopropyl, n-butyl, sec-butyl, tert-butyl, pentyl, hexyl, heptyl and the like; cyclic alkyl or cyclo alkyl include cyclo aliphatic, bicyclo aliphatic and tricycle aliphatic residues; examples of "cycloalkyl" include cyclopropyl, cyclobutyl, cyclopentyl, cyclohexyl, cycloheptyl, norbornyl and adamantyl;
- alkoxy: means alkyl-O, i.e. the radical obtained by removal of the oxygen-bound hydrogen from an aliphatic alcohol; (alkoxy)alkoxy refers to alkoxy groups, in which the alkyl group is substituted with one additional alkoxy group; examples of (alkoxy)alkoxy include methoxymethoxy with formula MeO-CH₂-O-, 2-(methoxy)ethoxy with formula MeO-CH₂-CH₂-O- and 2-(cyclopropylmethoxy)ethoxy with formula (C₃H₅)CH₂-O-CH₂-CH₂-O-;
- Ac: acetyl;
- tBu: tertiary butyl;
- cyanuric acid chloride: 2,4,6-trichloro-1,3,5-triazine
- dba: di(benzylidene)acetone
- DBU: 1,8-diazabicyclo[5.4.0]undec-7-ene;
- DABCO: 1,4-diazabicyclo[2.2.2]octane;
- DMF: N,N-dimethylformamide;
- DMA: N,N-dimethylacetamide;
- DMSO: dimethylsulfoxide;
- dppf: 1,1'-bis(diphenylphosphino)ferrocene
- halogen: means F, Cl, Br or J, preferably F, Cl or Br;
- hemiacetal: refers to the adduct of an alcohol, for instance methanol or ethanol, with a ketone or with an aldehyde; a hemiacetal may also result upon the addition of water to an enol ether; for instance, the hemiacetal of methanol with trifluoroacetone is F₃C-C(OH)(OCH₃)-CH₃;
- hexanes: mixture of isomeric hexanes;
- hydrate: refers to the adduct of water with a ketone or with an aldehyde, for instance, the hydrate of trifluoroacetone is F₃C-C(OH)₂-CH₃;
- LDA: Lithium diisopropyl amide
- NMP: N-methyl-2-pyrrolidone;
- sulfamic acid: HO-SO₂-NH₂;
- THF: tetrahydrofuran;
- trifluoroacetone: 1,1,1-trifluoropropan-2-one;
- xylene: 1,2-dimethylbenzene, 1,3-dimethylbenzene, 1,4-dimethylbenzene or a mixture thereof.

### SUMMARY OF THE INVENTION

Subject of the invention is a method for preparation of compound of formula (I); 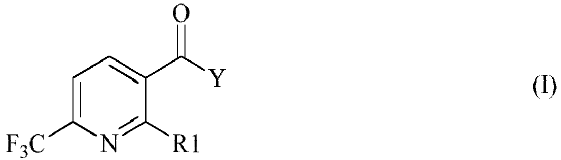 the method comprises a step (StepS1);
step (StepS1) comprises a reaction (ReacS1);
reaction (ReacS1) is a reaction of a compound of formula (II) with a compound (PhosS1) and a compound (HalS1) in the presence of a catalyst (CatS1); 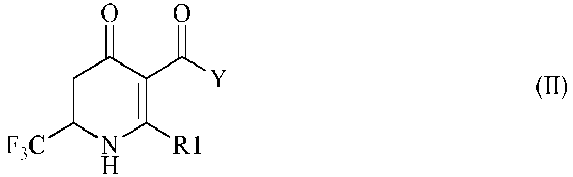 compound (PhosS1) is P(R11)(R12)(R13);
R11, R12 and R13 are identical or different and independently from each other selected from the group consisting of C₁₋₈ alkyl, C₃₋₁₀ cycloalkyl and phenyl, said phenyl being unsubstituted or substituted with 1 or 2 identical or different substituents independently from each other selected from the group consisting of C₁₋₆ alkyl and phenyl;
compound (HalS1) is selected from the group consisting of Cl₂, Br₂, I₂, ICl, BrCl, IBr, N-bromosuccinimide, N-chlorosuccinimide, N-bromophthalimide, N-chlorophthalimide, N,N',N"-trichloroisocyanuric acid, Ca(OCl)₂, CCl₄, CBr₄, hexachloroethane, 1,3-dibromo-5,5-dimethylhydantoin, 1,3-dichloro-5,5-dimethylhydantoin, diethyl azodicarboxylate, diisopropyl azodicarboxylate, azodicarbonamide, 1,1'-(azodicarbonyl)dipiperidine, and mixtures thereof;
catalyst (CatS1) is selected from the group consisting of elemental palladium, Pd(0) complex, Pd(II) complex, hydrate of Pd(0) complex, hydrate of Pd(II) complex, adduct of Pd(0) complex to solvent (SolvS1-Add) adduct of Pd(II) complex to solvent (SolvS1-Add), and mixtures thereof;
solvent (SolvS1-Add) is selected from the group consisting of dichloromethane, chloroform, acetonitrile, pyridine, toluene, benzene, and mixtures thereof;
R1 is selected from the group consisting of C₁₋₁₀ alkyl, C₃₋₈ cycloalkyl, C(O)-O-C₁₋₄ alkyl, CH=CH₂, benzyl, phenyl and naphthyl;
the C₁₋₁₀ alkyl of R1 is unsubstituted or substituted with 1, 2, 3, 4 or 5 identical or different substituents selected from the group consisting of halogen, OH, O-C(O)-C₁₋₅ alkyl, O-C₁₋₁₀ alkyl, S-C₁₋₁₀ alkyl, S(O)-C₁₋₁₀ alkyl, S(O₂)-C₁₋₁₀ alkyl, O-C₁₋₆ alkylen-O-C₁₋₆ alkyl, C₃₋₈ cycloalkyl and 1,2,4-triazolyl;
the benzyl, the phenyl and the naphthyl of R1 are independently from each other unsubstituted or substituted with 1, 2, 3, 4 or 5 identical or different substituents selected from the group consisting of halogen, C₁₋₄ alkoxy, NO₂ and CN;
Y is selected from the group consisting of C₁₋₆ alkoxy, O-C₁₋₆ alkylen-O-C₁₋₆ alkyl, NH₂, NHR4 and N(R4)R5;
R4 and R5 are identical or different and independently from each other C₁₋₆ alkyl, or represent together a tetramethylene or a pentamethylene chain.

### DETAILED DESCRIPTION OF THE INVENTION

Preferably, R11, R12, and R13 are identical or different and independently from each other selected from the group consisting of C₁₋₆ alkyl, C₃₋₇ cycloalkyl, and phenyl, said phenyl being unsubstituted or substituted with methyl or phenyl;
more preferably, R11, R12, and R13 are identical or different and independently from each other selected from the group consisting of C₁₋₄ alkyl, C₃₋₆ cycloalkyl, and phenyl, said phenyl unsubstituted or substituted with methyl;
especially, R11, R12, and R13 are identical or different and independently from each other selected from the group consisting of methyl, butyl, cyclohexyl, phenyl, and 2-tolyl;
Preferably, the molar ratio [compound (PhosS1) : compound of formula (II)] is from [20 : 1] to [1 : 1], more preferably from [10 : 1] to [1 : 1], even more preferably from [7.5 : 1] to [1 : 1], and especially from [5 : 1] to [1 : 1].
Preferably, compound (HalS1) is selected from the group consisting of Cl₂, Br₂, I₂, N-bromosuccinimide, N-chlorosuccinimide, N,N',N"-trichloroisocyanuric acid, CCl₄, CBr₄, 1,3-dibromo-5,5-dimethylhydantoin, 1,3-dichloro-5,5-dimethylhydantoin, azodicarbonamide, and mixtures thereof;
even more preferably, reagent (HalS1) is selected from the group consisting of Br₂, N-bromosuccinimide, CBr₄, 1,3-dibromo-5,5-dimethylhydantoin, and mixtures thereof;
especially, reagent (HalS1) is selected from the group consisting of Br₂, N-bromosuccinimide, 1,3-dibromo-5,5-dimethylhydantoin, and mixtures thereof.
Preferably, the molar ratio [compound (HalS1) : compound of formula (II)] is from [20 : 1] to [1 : 1], more preferably from [10 : 1] to [1 : 1], and even more preferably from [5 : 1] to [1 : 1].

Preferably, Pd(0) complex and Pd(II) complex are selected from the group consisting of PdCl₂, Pd(OAc)₂, Pd(dba)₂, Pd₂(dba)₃, PdCl₂(PhCN)₂, PdCl₂(MeCN)₂, PdCl₂(PPh₃)₂, Pd(P(tBu)₃)₂, Pd₂(P(tBu)₃)₃, Pd(P(1-adamantyl)₃)₂, Pd₂(P(1-adamantyl)₃)₃, Pd(PPh₃)₄, Pd(P(2-tolyl)₃)₄, PdCl₂(dppf), and mixtures thereof;
more preferably, Pd(0) complex and Pd(II) complex are selected from the group consisting of PdCl₂, Pd(OAc)₂, Pd(dba)₂, Pd₂(dba)₃, PdCl₂(PhCN)₂, PdCl₂(MeCN)₂, PdCl₂(PPh₃)₂, Pd(PPh₃)₄, Pd(P(2-tolyl)₃)₄, PdCl₂(dppf), and mixtures thereof.

Preferably, catalyst (CatS1) is selected from the group consisting of Pd(0), Pd(OAc)₂, PdCl₂, Pd₂(dba)₃, Pd(PPh₃)₄, Pd(P(2-tolyl)₃)₄, PdCl₂(dppf), hydrate of Pd(0) complex, hydrate of Pd(II) complex, adduct of Pd(0) complex to solvent (SolvS1-Add) adduct of Pd(II) complex to solvent (SolvS1-Add) and mixtures thereof;
more preferably, catalyst (CatS1) is selected from the group consisting of Pd(OAc)₂, PdCl₂, Pd(PPh₃)₄, adduct of Pd(0) complex to solvent (SolvS1-Add) adduct of Pd(II) complex to solvent (SolvS1-Add) and mixtures thereof.
Preferably, the molar ratio [catalyst (CatS1) : compound of formula (II)] is from [0.3 : 1] to [0.0001 : 1], more preferably from [0.15 : 1] to [0.001 : 1], even more preferably from [0.1 : 1] to [0.001 : 1], especially from [0.1 : 1] to [0.005 : 1], more especially from [0.1 : 1] to [0.01: 1].

Preferably, R1 is selected from the group consisting of C₁₋₅ alkyl, C₃₋₆ cycloalkyl, C(O)-O-C₁₋₄ alkyl, CH=CH₂, benzyl and phenyl;
said C₁₋₅ alkyl of R1 is unsubstituted or substituted with 1, 2, 3, 4 or 5 identical of different substituents selected from the group consisting of halogen, OH, O-C(O)-CH₃, O-C₁₋₅ alkyl, S-C₁₋₅ alkyl, S(O)-C₁₋₅ alkyl, S(O₂)-C₁₋₅ alkyl, O-C₁₋₄ alkylen-O-C₁₋₄ alkyl, C₃₋₆ cycloalkyl and 1,2,4-triazolyl;
said benzyl and said phenyl of R1 are independently from each other unsubstituted or substituted with 1, 2, 3, 4 or 5 identical of different substituents selected from the group consisting of halogen, C₁₋₂ alkoxy, NO₂ and CN;
more preferably, R1 is selected from the group consisting of methyl, ethyl, isopropyl, tert-butyl, cyclopropyl, trifluoromethyl, difluoromethyl, chloromethyl, bromomethyl, C(O)-O-CH₃, C(O)-O-C₂H₅, CH₂-O-C(O)-CH₃, CH₂- O-CH₃, CH₂-S-CH₃, CH₂-S(O₂)-CH₃, CH₂-CH₂- O-CH₃, CH₂-O-CH₂-CH₂-O-CH₃, CH₂-O-CH₂-CH₂-O-CH₂-CH₃, CH=CH₂ and phenyl.
even more preferably, R1 is selected from the group consisting of methyl, ethyl,
chloromethyl, bromomethyl, CH₂-O-C(O)-CH₃, and CH₂-O-CH₂-CH₂-O-CH₃; especially, R1 is selected from the group consisting of methyl, chloromethyl, and CH₂-O-CH₂-CH₂-O-CH₃.

Preferably, Y is selected from the group consisting of C₁₋₆ alkoxy, NHR4 and N(R4)R5; R4 and R5 are identical or different and independently from each other C₁₋₆ alkyl, or
represent together a tetramethylene or a pentamethylene chain; more preferably, Y is selected from the group consisting of methoxy and ethoxy.
Reaction (ReacS1) can be done in the presence of a base (BasS1); base (BasS1) is selected from the group consisting of N,N-di-C₁₋₄ alkyl aniline, N(R40)(R41)(R42), N-C₁₋₄ alkyl morpholine, unsubstituted pyridine, pyridine substituted with 1, 2 and 3 identical or different substituents independently from each other selected from the group consisting of methyl and ethyl, and mixtures thereof;
R40, R41 and R42 are identical or different and independently from each other C₁₋₆ alkyl; more preferably, base (BasS1) is selected from the group consisting of N,N-dimethylaniline, triethylamine, diisopropylethylamine, N-methylmorpholine, N-ethylmorpholine, pyridine, 2-methylpyridine, 3-methylpyridine, 4-methylpyridine, 2-methyl-5-ethylpyridine, 2,4,6-trimethylpyridine, and mixtures thereof;
even more preferably, base (BasS1) is selected from the group consisting of pyridine, 3-methylpyridine, 2-methyl-5-ethylpyridine, 2,4,6-trimethylpyridine, N-methylmorpholine, N-ethylmorpholine, and mixtures thereof;
especially, base (BasS1) is selected from the group consisting of pyridine, 3-methylpyridine, N-methylmorpholine, N-ethylmorpholine, and mixtures thereof;
more especially, base (BasS1) is selected from the group consisting of pyridine, 3-methylpyridine, N-methylmorpholine, and mixtures thereof.
Preferably, the molar ratio [base (BasS1) : compound of formula (II)] is from [500 : 1] to [0.1 : 1], more preferably from [200 : 1] to [0.5 : 1], even more preferably from [100 : 1] to [1 : 1]; especially preferably from [75 : 1] to [1 : 1].

Reaction (ReacS1) can be done in a solvent (SolvS1);
solvent (SolvS1) is selected from the group consisting of THF, 2-methyl-THF, 3-methyl-THF, DMF, DMA, acetonitrile, propionitrile, 1,2-dimethoxyethane, methoxycyclopentane, diethyleneglycoldimethylether, diethyleneglycoldiethylether, benzene, toluene, chlorobenzene, anisole, pyridine, 3-methylpyridine, N-methylmorpholine, N-ethylmorpholine, dichloromethane, chloroform, and mixtures thereof;
preferably, solvent (SolvS1) is selected from the group consisting of THF, 2-methyl-THF, 3-methyl-THF, acetonitrile, 1,2-dimethoxyethane, benzene, toluene, anisole, pyridine, 3-methylpyridine, N-methylmorpholine, N-ethylmorpholine, dichloromethane, and mixtures thereof;
more preferably, solvent (SolvS1) is selected from the group consisting of THF, 2-methyl-THF, 3-methyl-THF, acetonitrile, 1,2-dimethoxyethane, toluene, pyridine, 3-methylpyridine, N-methylmorpholine, N-ethylmorpholine, dichloromethane, and mixtures thereof.
Preferably, the weight of solvent (SolvS1) is from 0.1 to 100 times, more preferably from 1 to 50 times, even more preferably from 1 to 25 times, of the weight of compound of formula (II).

Preferably, the reaction temperature of reaction (ReacS1) is from -70 to 150°C, more preferably from -50 to 120°C, even more preferably from -40 to 100°C, especially from -25 to 100°C.

Preferably, reaction (ReacS1) is done at a pressure of from ambient pressure to 20 bar, more preferably of from ambient pressure to 15 bar, even more preferably of from ambient pressure to 10 bar.
Preferably, the reaction time of reaction (ReacS1) is from 30 min to 96 h, more preferably from 1 h to 48 h, even more preferably from 2 h to 36 h.

After reaction (ReacS1), compound of formula (I) can be isolated by any conventional method.
Preferably, compound of formula (I) is isolated after reaction (ReacS1) by hydrolysis and acidification of the reaction mixture resulting from reaction (ReacS1).
Hydrolysis and acidification is preferably done by addition of a compound (InAcS1), compound (InAcS1) is an aqueous inorganic acid, preferably compound (InAcS1) is selected from the group consisting of aqueous hydrochloric acid and aqueous sulfuric acid.
After hydrolysis and acidification, any solvent (SolvS1) is preferably removed by distillation; compound of formula (I) is preferably extracted by extraction with a solvent (SolvExtrS1), solvent (SolvExtrS1) is preferably selected from the group consisting of ethyl acetate, isopropyl acetate, butyl acetate, toluene, chlorobenzene, dichloromethane, chloroform and mixtures thereof; the extraction is preferably followed by removal of solvent (SolvExtrS1) by distillation.

Also possible is the purification of compound of formula (I) by saponification by treatment of the reaction mixture resulting from reaction (ReacS1) or by treatment of the crude product of formula (I) with an aqueous or methanolic solution of NaOH or KOH, followed by acidification and extraction with solvent (SolvExtrS1), preferably followed by removal of solvent (SolvExtrS1) by distillation, to yield compound of formula (I) with Y = OH.

Preferably, the method comprises furthermore a step (S2), step (S2) is done before step (S1), step (StepS2) comprises a reaction (ReacS2), in reaction (ReacS2) compound of formula (II) is prepared;
reaction (ReacS2) is a reaction of a compound of formula (III) with a base (BasS2); 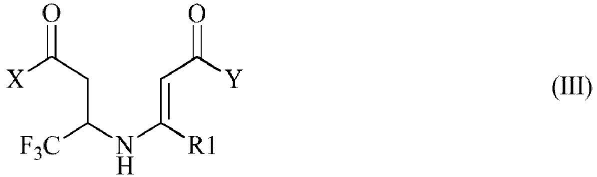 X is C₁₋₆ alkoxy or phenoxy;
base (BasS2) is selected from the group consisting of NaOC₁₋₆ alkyl, KOC₁₋₆ alkyl, NaN(SiMe₃)₂, KN(SiMe₃)₂, NaH, LiH, CaH₂, KH, and mixtures thereof;
with compound of formula (II), R1 and Y as defined above, also with all their preferred embodiments.

Preferably, X is C₁₋₄ alkoxy;
more preferably, X is methoxy or ethoxy.

Preferably, base (BasS2) is selected from the group consisting of NaOtBu, KOtBu, NaOEt, NaOMe, NaOiPr, NaN(SiMe₃)₂, KN(SiMe₃)₂, NaH, LiH, CaH₂, KH, and mixtures thereof;
more preferably, base (BasS2) is selected from the group consisting of NaOtBu, KOtBu, NaOEt, NaOMe, NaN(SiMe₃)₂, NaH, KH, and mixtures thereof.
Preferably, the molar ratio [base (BasS2) : compound of formula (III)] is from [20 : 1] to [1.9 : 1], more preferably from [10 : 1] to [1.9 : 1], and even more preferably from [5 : 1] to [1.9 : 1].

Preferably, reaction (ReacS2) is done in a solvent (SolvS2);
solvent (SolvS2) is selected from the group of C₁₋₆ alkanol, acetonitrile, propionitrile, benzene, toluene, xylene, chlorobenzene, anisole, pyridine, 2-methylpyridine, 3-methylpyridine, 4-methylpyridine, 2-methyl-5-ethylpyridine, DMF, DMA, DMSO, THF, 2-methyl-THF, 3-methyl-THF, 1,2-dimethoxyethane, diethyleneglycoldimethylether, and mixtures thereof;
more preferably, solvent (SolvS2) is selected from the group consisting of C₄-₆ alkanol, acetonitrile, propionitrile, toluene, xylene, chlorobenzene, 3-methylpyridine, DMF, DMA, THF, 2-methyl-THF, 3-methyl-THF, 1,2-dimethoxyethane, diethyleneglycoldimethylether, and mixtures thereof.
Preferably, the weight of solvent (SolvS2) is from 0.1 to 100 times, more preferably from 1 to 50 times, even more preferably from 1 to 20 times, of the weight of compound of formula (III).

Preferably, reaction (ReacS2) is done at a temperature of from -50 to 200°C, more preferably from -30 to 150°C, even more preferably from -10 to 130°C, especially from -10 to 100°C, more especially from -10 to 80°C.
Preferably, reaction (ReacS2) is done at a pressure of from 0.5 to 20 bar, more preferably from ambient pressure to 10 bar, even more preferably from ambient pressure to 5 bar.
Preferably, reaction time of reaction (ReacS2) is from 10 min to 72 h, more preferably from 1 h to 48 h, even more preferably from 2 h to 36 h.

After reaction (ReacS2), compound of formula (II) may be isolated by any conventional method.
Preferably, the reaction mixture is added to dilute aqueous acid, such as dilute aqueous hydrochloric or sulfuric acid, any solvent is distilled off, and the product of formula (II) is either isolated by filtration or extracted with a solvent (SolvExtrS2), solvent (SolvExtrS2) is preferably selected from the group consisting of ethyl acetate, isopropyl acetate, butyl acetate, toluene, chlorobenzene, dichloromethane, chloroform, and mixtures thereof; after distilling off solvent (SolvExtrS2), the crude product may be further purified by recrystallization.

Preferably, the method comprises furthermore a step (S3), step (S3) is done before step (S2); step (StepS3) comprises a reaction (ReacS3), in reaction (ReacS3) compound of formula (III) is prepared;
reaction (ReacS3) is a reaction of a compound of formula (IV) with a compound of formula (V); 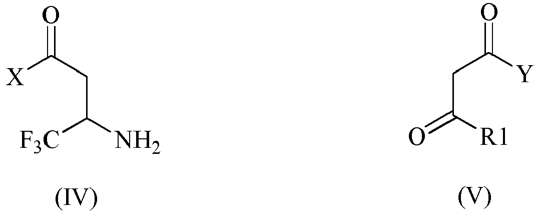 with compound of formula (III), X, R1 and Y as defined above, also with all their preferred embodiments.

Preferably, the molar ratio [compound of formula (IV) : compound of formula (V)] is from [5 : 1] to [1 : 5], more preferably from [3 : 1] to [1 : 3], and even more preferably from [1.5 : 1.0] to [1.0 : 1.5].
Compound of formula (IV) and compound of formula (V) can be used in equimolar ratio, but each of the two compounds can also be used in excess with respect to the other one. How the ratio is chosen depends also on the price of each compound.

Reaction (ReacS3) can be done in a solvent (SolvS3);
solvent (SolvS3) is selected from the group of C₁₋₆ alkanol, acetonitrile, propionitrile, benzene, toluene, chlorobenzene, dichloromethane, chloroform, tetrahydrofuran, 2-methyltetrahydrofuran, 3-methyltetrahydrofuran, 1,2-dimethoxyethane, methoxycyclopentane, and mixtures thereof;
preferably, solvent (SolvS3) is selected from the group consisting of C₁₋₆ alkanol, acetonitrile, toluene, dichloromethane, tetrahydrofuran, 2-methyltetrahydrofuran, 3-methyltetrahydrofuran, 1,2-dimethoxyethane, and mixtures thereof;
more preferably, solvent (SolvS3) is selected from the group consisting of C₁₋₆ alkanol, acetonitrile, toluene, dichloromethane, tetrahydrofuran, 1,2-dimethoxyethane, and mixtures thereof.
Preferably, the weight of solvent (SolvS3) is from 100 to 0.1 times, more preferably from 50 to 0.2 times, even more preferably from 30 to 0.3 times, of the weight of compound of formula (IV).

Preferably, reaction (ReacS3) is done at a temperature of from 0 to 160 °C, more preferably from 10 to 140 °C, even more preferably from 15 to 120 °C.

Preferably, reaction (ReacS3) is done at a pressure of from 0.2 to 20 bar, more preferably from 0.5 to 10 bar, even more preferably from 0.7 to 5 bar.
Preferably, reaction time of reaction (ReacS3) is from 5 min to 48 h, more preferably from 0.2 h to 24 h, even more preferably from 0.5 h to 12 h.

Reaction (ReacS3) can be conducted at the reflux temperature of a solvent (SolvS3) or solvent mixture capable of forming an azeotrope with water, in order to remove the water formed during the reaction from the reaction mixture.

After reaction (ReacS3), compound of formula (III) may be isolated by any conventional method.
Preferably, any solvent (SolvS3) is partly or completely removed by distillation, and the product of formula (III) is used without further purification.
In a another preferable embodiment, the reaction mixture resulting from reaction (ReacS3) is used directly for reaction (ReacS2).

Compound of formula (IV) and compound of formula (V) are known compounds and can be prepared according to or in analogy to known methods or can be purchased on the market.

The compound of formula (II), compound of formula (III), compound of formula (IV) and compound of formula (V) comprise also any of the possible tautomeric forms.

Further subject of the invention are compound of formula (III) and compound of formula (II); wherein compound of formula (III) and compound of formula (II) are defined as above, also with all their preferred embodiments.

Further subject of the invention are compound of formula (III) and compound of formula (II); wherein
compound of formula (III) is compound of formula (III-1); and
compound of formula (II) is compound of formula (II-1).

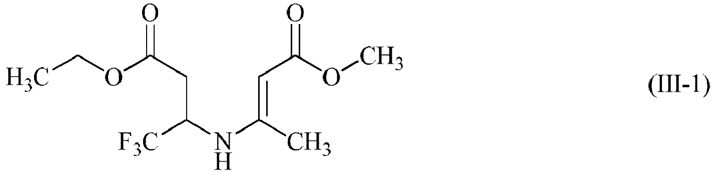

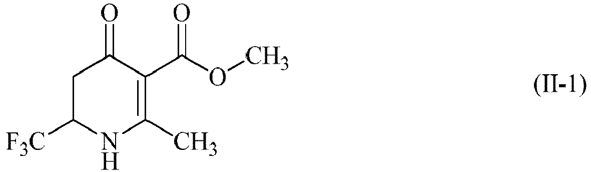

Further subj ect of the invention is the use of any of the compound of formula (III), compound of formula (II) and compound of formula (I) for the production of pharmaceutical, chemical or agro-chemical products,
with compound of formula (III), compound of formula (II) and compound of formula (I) as defined above, also with all their preferred embodiments,
wherein any of the compound of formula (III), compound of formula (II) and compound of formula (I) has been prepared according to one of the respective methods as described above, also with all their preferred respective embodiments.

### Examples

### Example 1: Reaction (ReacS3)

To a solution of racemic ethyl 4,4,4-trifluoro-3-aminobutanoate (approx 20 mmol; prepared by hydrogenation of ethyl 4,4,4-trifluoro-3-aminocrotonate, in analogy to Cen et al. Journal of Fluorine Chemistry 1995, 73, 161-164) in acetonitrile (3 ml) methyl acetoacetate (3.11 ml, 29 mmol) was added. The mixture was stirred at room temperature for 4 h. The mixture was concentrated under reduced pressure (40 mbar, 60 °C, 15 min), and the NMR of the residue showed compound of formula (III-1).

¹H NMR (400 MHz, d₆-DMSO): delta 1.18 (t, J = 7 Hz, 3H), 2.00 (s, 3H), 2.76 (dd, J = 16 Hz, 7 Hz, 1H), 2.96 (dd, J = 16 Hz, 2 Hz, 1H), 3.53 (s, 3H), 4.11 (q, J = 7 Hz, 2H), 4.60 (s, 1H), 4.75 (m, 1H), 8.70 (d, J = 9 Hz, 1H).

### Example 2: Reaction (ReacS2)

Compound of formula (III-1), prepared according to example 1 and used without further purification, was dissolved in THF (20 ml), and this solution was added drop wise to a suspension of NaOtBu (6.6 g, 69 mmol) in THF (30 ml), while keeping the temperature at 20 to 40 °C with an ice bath. The resulting mixture was stirred at room temperature for 17 h. The mixture was then poured onto aqueous hydrochloric acid (1 N, 100 ml). The THF was removed by distillation under reduced pressure, and the remaining aqueous solution was saturated with sodium chloride. Extraction (3 x 30 ml AcOEt), drying of the combined extracts with MgSO₄, and concentration under reduced pressure yielded 4.59 g of compound of formula (II-1) as an oil, which crystallized completely within a few days.

¹H NMR (400 MHz, d₆-DMSO): delta 2.19 (s, 3H), 2.35 (dd, J = 16 Hz, 1 Hz, 1H), 2.89 (dd, J = 16 Hz, 7 Hz, 1H), 3.59 (s, 3H), 4.54 (m, 1H), 8.80 (br s, 1H).

### Example 3: Reaction (ReacS1)

To a solution of compound of formula (II-1) (50 mg, 0.21 mmol, prepared according to example 2 and used without further purification) in pyridine (1 ml, 12.4 mmol) were added Pd(OAc)₂ (5 mg, 0.02 mmol), PPh₃ (145 mg, 0.55 mmol), and finally bromine (0.022 ml, 0.4 mmol). The resulting mixture was stirred at 80 °C for 18 h. The mixture was diluted with aqueous hydrochloric acid (1 N, 15 ml) and extracted with AcOEt (3 ml). A sample of this extract was concentrated and analyzed. ¹H NMR indicated it to be a mixture of compound of formula (I-1) and unchanged starting material, i.e. compound of formula (II-1), in a molar ratio of 60 : 40, and triphenylphosphine oxide.

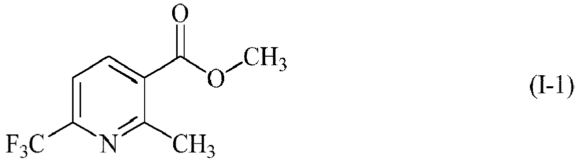

¹H NMR (400 MHz, d₆-DMSO): delta 2.78 (s, 3H), 3.91 (s, 3H), 7.89 (d, J = 8 Hz, 1H), 8.44 (d, J = 8 Hz, 1H).

### Example 4: Reaction (ReacS1)

### Preparation of the a catalyst (CatS1-1):

To Pd(OAc)₂ (16 mg, 0.07 mmol) in pyridine (0.5 ml) was added PPh₃ (74 mg, 0.28 mmol), and the mixture was heated to reflux for 30 s, providing catalyst (CatS1-1) in form of a solution.

### Reaction (Reac(S1):

To a solution of compound of formula (II-1) (50 mg, 0.21 mmol, prepared according to example 2 and used without further purification) in acetonitrile (0.5 ml) were added pyridine (0.17 ml, 2.1 mmol), PPh₃ (173 mg, 0.66 mmol), catalyst (CatS1-1) (0.08 ml, corresponds to 0.011 mmol Pd complex), and finally bromine (0.032 ml, 0.63 mmol). The resulting mixture was stirred at 80 °C for 21 h. The mixture was diluted with aqueous hydrochloric acid (1 N, 15 ml) and extracted with AcOEt (3 ml). A sample of this extract was concentrated and analyzed. ¹H NMR indicated it to be a mixture of compound of formula (I-1) and unchanged starting material, i.e. compound of formula (II-1), in a molar ratio of 75 : 25, and triphenylphosphine oxide.

### Example 5: Reaction (ReacS1) and saponification of the ester

### Preparation of a catalyst (CatS1-2):

To Pd(OAc)₂ (16 mg, 0.07 mmol) in pyridine (0.5 ml) was added PPh₃ (61 mg, 0.23 mmol), and the mixture was heated to reflux for 30 s, providing catalyst (CatS1-2) in form of a solution.

### Reaction (ReacS1) and saponification of the ester:

To a solution of compound of formula (II-1) (70%, 156 mg, 0.46 mmol, prepared according to example 2 and used without further purification) in acetonitrile (1.5 ml) were added pyridine (0.51 ml, 6.6 mmol), PPh₃ (173 mg, 0.66 mmol), catalyst (CatS1-2) (0.24 ml, corresponds to 0.034 mmol Pd complex), and finally, at 0 °C, bromine (0.097 ml, 1.9 mmol). The resulting mixture was stirred at 80 °C for 18 h. Then a solution of NaOH (0.36 g, 9 mmol) in water (0.75 ml) was added, and stirring at 80 °C was continued for 1.5 h. The mixture was diluted with brine (15 ml) and washed with toluene (3 x 5 ml). The aqueous phase was then acidified with aqueous concentrated hydrochloric acid to pH 2, and extracted with AcOEt (3 x 10 ml). The combined extracts were dried (MgSO₄) and concentrated under reduced pressure to yield 40 mg (42%) of the compound of formula (I-Sap-1).

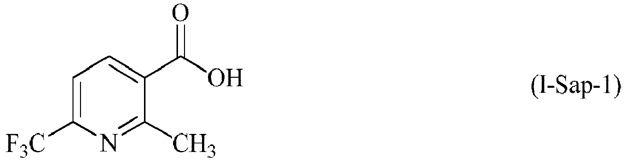

¹H NMR (400 MHz, d₆-DMSO): delta 2.78 (s, 3H), 7.85 (d, J = 8 Hz, 1H), 8.41 (d, J = 8 Hz, 1H).

## Claims

1. Method for preparation of compound of formula (I); 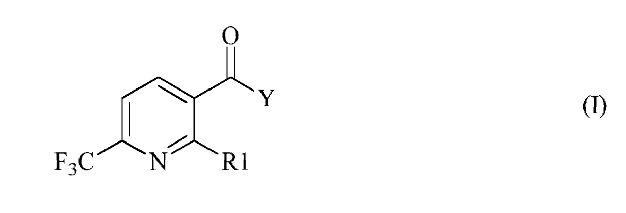 the method comprises a step (StepS1);
step (StepS1) comprises a reaction (ReacS1);
reaction (ReacS1) is a reaction of a compound of formula (II) with a compound (PhosS1) and a compound (HalS1) in the presence of a catalyst (CatS1); 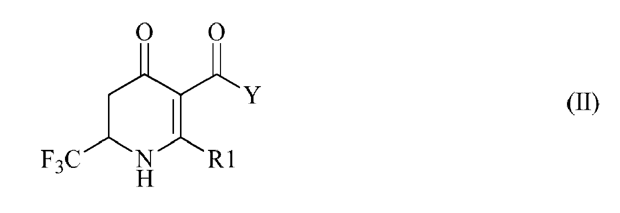 compound (PhosS1) is P(R11)(R12)(R13);
R11, R12 and R13 are identical or different and independently from each other selected from the group consisting of C₁₋₈ alkyl, C₃₋₁₀ cycloalkyl and phenyl,
said phenyl being unsubstituted or substituted with 1 or 2 identical or different substituents independently from each other selected from the group consisting of C₁₋₆ alkyl and phenyl;
compound (HalS1) is selected from the group consisting of Cl₂, Br₂, I₂, ICl, BrCl, IBr, N-bromosuccinimide, N-chlorosuccinimide, N-bromophthalimide, N-chlorophthalimide, N,N',N"-trichloroisocyanuric acid, Ca(OCl)₂, CCl₄, CBr₄, hexachloroethane, 1,3-dibromo-5,5-dimethylhydantoin, 1,3-dichloro-5,5-dimethylhydantoin, diethyl azodicarboxylate, diisopropyl azodicarboxylate, azodicarbonamide, 1,1'-(azodicarbonyl)dipiperidine, and mixtures thereof;
catalyst (CatS1) is selected from the group consisting of elemental palladium, Pd(0) complex, Pd(II) complex, hydrate of Pd(0) complex, hydrate of Pd(II) complex, adduct of Pd(0) complex to solvent (SolvS1-Add) adduct of Pd(II) complex to solvent (SolvS1-Add), and mixtures thereof;
solvent (SolvS1-Add) is selected from the group consisting of dichloromethane, chloroform, acetonitrile, pyridine, toluene, benzene, and mixtures thereof;
R1 is selected from the group consisting of C₁₋₁₀ alkyl, C₃₋₈ cycloalkyl, C(O)-O-C₁₋₄ alkyl, CH=CH₂, benzyl, phenyl and naphthyl;
the C₁₋₁₀ alkyl of R1 is unsubstituted or substituted with 1, 2, 3, 4 or 5 identical or different substituents selected from the group consisting of halogen, OH, O-C(O)-C₁₋₅ alkyl, O-C₁₋₁₀ alkyl, S-C₁₋₁₀ alkyl, S(O)-C₁₋₁₀ alkyl, S(O₂)-C₁₋₁₀ alkyl, O-C₁₋₆ alkylen-O-C₁₋₆ alkyl, C₃₋₈ cycloalkyl and 1,2,4-triazolyl;
the benzyl, the phenyl and the naphthyl of R1 are independently from each other unsubstituted or substituted with 1, 2, 3, 4 or 5 identical or different substituents selected from the group consisting of halogen, C₁₋₄ alkoxy, NO₂ and CN;
Y is selected from the group consisting of C₁₋₆ alkoxy, O-C₁₋₆ alkylen-O-C₁₋₆ alkyl, NH₂, NHR4 and N(R4)R5;
R4 and R5 are identical or different and independently from each other C₁₋₆ alkyl, or represent together a tetramethylene or a pentamethylene chain.

2. Method according to claim1, wherein
R11, R12, and R13 are identical or different and independently from each other selected from the group consisting of C₁₋₆ alkyl, C₃₋₇ cycloalkyl, and phenyl, said phenyl being unsubstituted or substituted with methyl or phenyl.

3. Method according to claim 1 or 2, wherein
compound (HalS1) is selected from the group consisting of Cl₂, Br₂, I₂, N-bromosuccinimide, N-chlorosuccinimide, N,N',N"-trichloroisocyanuric acid, CCl₄, CBr₄, 1,3-dibromo-5,5-dimethylhydantoin, 1,3-dichloro-5,5-dimethylhydantoin, azodicarbonamide, and mixtures thereof.

4. Method according to one or more of claims 1 to 3, wherein
Pd(0) complex and Pd(II) complex are selected from the group consisting of PdCl₂, Pd(OAc)₂, Pd(dba)₂, Pd₂(dba)₃, PdCl₂(PhCN)₂, PdCl₂(MeCN)₂, PdCl₂(PPh₃)₂, Pd(P(tBu)₃)₂, Pd₂(P(tBu)₃)₃, Pd(P(1-adamantyl)₃)₂, Pd₂(P(1-adamantyl)₃)₃, Pd(PPh₃)₄, Pd(P(2-tolyl)₃)₄, PdCl₂(dppf), and mixtures thereof.

5. Method according to one or more of claims 1 to 4, wherein
catalyst (CatS1) is selected from the group consisting of Pd(0), Pd(OAc)₂, PdCl₂, Pd₂(dba)₃, Pd(PPh₃)₄, Pd(P(2-tolyl)₃)₄, PdCl₂(dppf), hydrate of Pd(0) complex, hydrate of Pd(II) complex, adduct of Pd(0) complex to solvent (SolvS1-Add) adduct of Pd(II) complex to solvent (SolvS1-Add) and mixtures thereof.

6. Method according to one or more of claims 1 to 5, wherein
R1 is selected from the group consisting of C₁₋₅ alkyl, C₃₋₆ cycloalkyl, C(O)-O-C₁₋₄ alkyl, CH=CH₂, benzyl and phenyl;
said C₁₋₅ alkyl of R1 is unsubstituted or substituted with 1, 2, 3, 4 or 5 identical of different substituents selected from the group consisting of halogen, OH, O-C(O)-CH₃, O-C₁₋₅ alkyl, S-C₁₋₅ alkyl, S(O)-C₁₋₅ alkyl, S(O₂)-C₁₋₅ alkyl, O-C₁₋₄ alkylen-O-C₁₋₄ alkyl, C₃₋₆ cycloalkyl and 1,2,4-triazolyl;
said benzyl and said phenyl of R1 are independently from each other unsubstituted or substituted with 1, 2, 3, 4 or 5 identical of different substituents selected from the group consisting of halogen, C₁₋₂ alkoxy, NO₂ and CN.

7. Method according to one or more of claims 1 to 6, wherein
Y is selected from the group consisting of C₁₋₆ alkoxy, NHR4 and N(R4)R5;
R4 and R5 are identical or different and independently from each other C₁₋₆ alkyl, or represent together a tetramethylene or a pentamethylene chain.

8. Method according to one or more of claims 1 to 7, wherein
reaction (ReacS1) is done in the presence of a base (BasS1); base (BasS1) is selected from the group consisting of N,N-di-C₁₋₄ alkyl aniline, N(R40)(R41)(R42), N-C₁₋₄ alkyl morpholine, unsubstituted pyridine, pyridine substituted with 1, 2 and 3 identical or different substituents independently from each other selected from the group consisting of methyl and ethyl, and mixtures thereof;
R40, R41 and R42 are identical or different and independently from each other C₁₋₆ alkyl.

9. Method according to one or more of claims 1 to 8, wherein
the method comprises furthermore a step (S2), step (S2) is done before step (S1);
step (StepS2) comprises a reaction (ReacS2), in reaction (ReacS2) compound of formula (II) is prepared;
reaction (ReacS2) is a reaction of a compound of formula (III) with a base (BasS2); 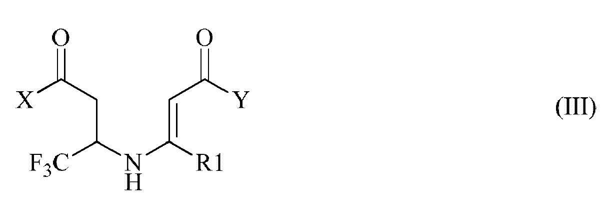 X is C₁₋₆ alkoxy or phenoxy;
base (BasS2) is selected from the group consisting of NaOC₁₋₆ alkyl, KOC₁₋₆ alkyl, NaN(SiMe₃)₂, KN(SiMe₃)₂, NaH, LiH, CaH₂, KH, and mixtures thereof;
with compound of formula (II), R1 and Y as defined in claim 1.

10. Method according to claim 9, wherein
X is C₁₋₄ alkoxy.

11. Method according to claim 9 or 10, wherein
base (BasS2) is selected from the group consisting of NaOtBu, KOtBu, NaOEt, NaOMe, NaOiPr, NaN(SiMe₃)₂, KN(SiMe₃)₂, NaH, LiH, CaH₂, KH, and mixtures thereof.

12. Method according to one or more of claims 9 to 11, wherein
the method comprises furthermore a step (S3), step (S3) is done before step (S2);
step (StepS3) comprises a reaction (ReacS3), in reaction (ReacS3) compound of formula (III) is prepared;
reaction (ReacS3) is a reaction of a compound of formula (IV) with a compound of formula (V); 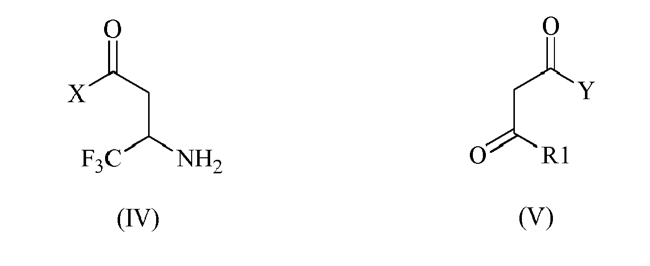 with compound of formula (III) and X as defined in claim 9, and with R1 and Y as defined in claim 1.

13. Compound of formula (III) and compound of formula (II); wherein
compound of formula (III) is as defined in claim 9;
and compound of formula (II) is as defined in claim 1.

14. Compound of formula (III) and compound of formula (II) according to claim 13, wherein
compound of formula (III) is compound of formula (III-1); and
compound of formula (II) is compound of formula (II-1).

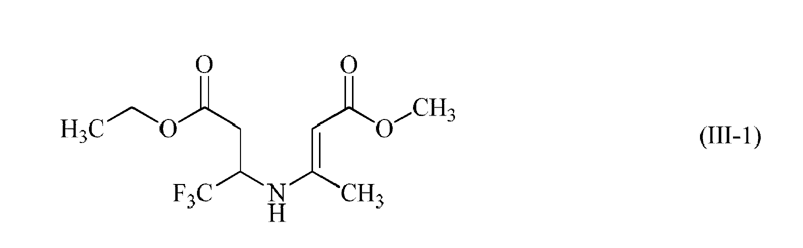

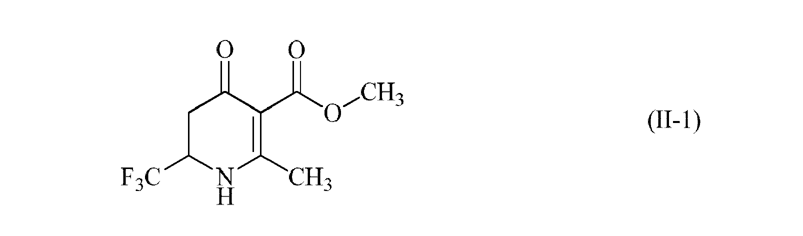

15. Use of any of the compound of formula (III), compound of formula (II) and compound of
formula (I) for the production of pharmaceutical, chemical or agro-chemical products, with compound of formula (III) as defined in claim 9, and with compound of formula (II) and
compound of formula (I) as defined in claim 1,
wherein
compound of formula (III) has been prepared according to claim 12,
compound of formula (II) has been prepared according to claim 9, and compound of formula (I) has been prepared according to claim 1.
